# EUROPEAN PATENT SPECIFICATION

(11) **EP 1 294 840 B1**
(45) Date of publication and mention of the grant of the patent: **26.01.2011**
(21) Application number: 01936616.0
(22) Date of filing: 23.05.2001
(51) Int. Cl.: C11D 3/12, C01B 39/00, D21H 17/67, D21H 17/68

(54) **ZEOLITE COMPOSITIONS AND THEIR USE**
ZEOLITHZUSAMMENSETZUNGEN UND IHRE ANWENDUNG
COMPOSITIONS A BASE DE ZEOLITE ET LEUR UTILISATION

(30) Priority: 02.06.2000 GB 0013406
(43) Date of publication of application: 26.03.2003
(73) Proprietor: PQ Silicas UK Limited, Cheshire WA5 1AQ (GB)
(72) Inventor: ARAYA, Abraham, Wirral, Merseyside L63 3JX (GB)
(74) Representative: Collingwood, Anthony Robert
(86) International application number: PCT/GB2001/002305
(87) International publication number: WO 2001/094512

(56) References cited:
- EP-A- 0 384 070
- EP-A- 0 565 364

## Description

This invention relates to zeolite compositions and their use and, in particular, to zeolite compositions which are used in a medium having a pH value well below the natural pH value of zeolite slurries.

Zeolites, which are crystalline aluminosilicates, are well-known materials having a variety of uses, and are frequently described as molecular sieves. An aqueous slurry of some zeolites, particularly those with a low Si to Al ratio have an alkaline pH, often greater than 10. However, there is a number of uses where it would be convenient if such zeolite slurries could be produced at a lower pH. These uses include paper making, paints, dental applications, low pH detergents, for fabrics and dishes, and certain adsorption and catalytic applications. Any attempt to reduce the pH of an alkaline slurry of such zeolites to a value in the range of, say, 5 to 9, by, for example, the addition of acid, usually results, in time, in the breakdown of the crystalline nature of the zeolite due to removal of aluminium species by hydrolysis. Moreover, addition of large volumes of water is also+ineffective in lowering the pH of a zeolite slurry.

US4874504 discloses improved control over the noble-metal exchange of zeolite catalyst by using bulky organic bases to control pH during the metal loading step. The pH of an aqueous suspension of zeolite is adjusted with an organic base of sufficient size to prevent entry of organic cations into the pore structure of the zeolite caltalyst. The disclosure is concerned with materials of desser acid strength than conventional aluminosilicate zeolites, for example Ga - ad In - Zeolites.

Surprisingly, a zeolite composition has now been devised which makes available an aqueous slurry of zeollie which is stable at a pH value well below the value at which known slurries of zeolites are stable and which can be used for a variety of applications utilising a pH lower than that possible with conventional formulations containing zeolites. Using these compositions it is possible to produce an aqueous slurry of zeolite with a pH well below 10, without the addition of an acid.

In general, the empirical formula of a zeolite is

M_{2/n}O · Al₂O₃ · xSiO₂ · yH₂O

wherein M represents a metallic cation having a valency of n, x indicates the ratio of molecules of SiO₂ to mefeautes of Al₂O₃ and y indicates the ratio of molecules of water to molecules of Al₂O₃. Many different types of zeolite, with varying ratios of silica to alumina, are known. Commonly, however, M is an alkali metal.

According to one aspect of this invention, a method of using a zeolite composition preparing an aqueous composition with a pH in the range 4-10 comprising forming a mixture of
(a) a crystalline aluminosilicate represented by the empirical formula

   M_{2/n}O · Al₂O₃ · xSiO₂ · yH₂O

   wherein M represents a first metal moiety, said first metal having a valency of n, x indicates the ratio of molecules of SiO₂ to molecules of Al₂O₃ and y indicates the ratio of molecules of water to molecules of Al₂O₃ molecules of Al₂O₃ and
(b) a salt of a second metal selected from the group consisting of Group III metals, metallic elements of Group IV, magnesium, titanium, chromium, iron, nickel, copper, zinc, zirconium and silver, said salt of a second metal being present in an amount which is sufficient to replace from about 2.0 to about 40 per cent by weight of the first metal moiety,
   and using said mixture in the aqueous composition characterised in that mis an alkali metal

The above form of empirical formula is used for simplicity in expressing the molar ratios of the components, but it can be seen that the ratio of Si atoms to Al atoms in this formula is equal to x/2 and the ratio of water molecules to Al atoms is equal to y/2.

The preferred alkali metal is sodium.

The zeolites used in the invention may have the structure of any of the known zeolites. The structure and characteristics of many zeolites are described in the standard work "Zeolite Molecular Sieves" by Donald W. Breck, published by Robert E, Krieger Publishing Company. Usually, the value of x in the above empirical formula is in the range 1.5 to 10. The value of y, which represents the amount of water contained in the voids of the zeolite, can vary widely. In anhydrous material y = 0 and, in fully hydrated zeolites, y is typically up to 5.

Zeolites useful in this invention may be based on naturally-occurring or synthetic aluminosilicates and the preferred forms of zeolite have the structure known as zeolite P, zeolite X or zeolite A. Particularly preferred forms of zeolite are those disclosed in EP-A-0 384 070, EP-A-0 565 364, EP-A-0 697 010. EP-A-0 742 780, WO-A-96/14270, WO-A-96/34828 and WO-A- 97/06102. The zeolite P described in EP-A-0 384 070 has the empirical formula given above in which M represents an alkali metal and x has a value up to 2.66, preferably in the range 1.8 to 2.66, and has a structure which is particularly useful in the present invention.

The preferred amount of the salt of a second metal used to prepare mixtures used in the invention depends upon a number of factors, such as the actual second metal selected, the actual zeolite chosen and the desired effect to be achieved (for example, the pH at which the mixture is used). Useful mixtures contain an amount of second metal salt sufficient to replace from 3.0 to 30 per cent by weight of first metal, and, more preferably, from 3.0 to 20 per cent by weight of the first metal.

The appropriate amount of salt of a second metal in the mixture used in the invention, expressed in grams, depends upon the composition of the zeolite and the charge on the second metal but is readily calculated by a skilled person. For example, a zeolite having the empirical formula

Na₂O · Al₂O₃ · 2SiO₂

(anhydrous type A zeolite) contains 16.2 g of Na per 100 g of zeolite. An aluminium ion is capable of replacing three sodium ions. Hence, making allowance for the atomic weights of sodium and aluminium, it can be calculated that a mixture containing 100 g of anhydrous zeolite A and 0.317 g Al in the form of a suitable salt is a mixture suitable for use in the invention and containing sufficient salt of a second metal (i.e. aluminium) to replace 5 per cent by weight of the first metal moiety (i.e. sodium).

It has been observed that a salt of a second metal wherein the ion of the second metal has a relatively small size or a relatively high charge is more effective in stabilising a zeolite slurry at a low pH. Consequently, the observed stability at low pH is more marked when the salt of a second metal is a salt of aluminium than when it is a salt of magnesium and the effect is more marked for salts of magnesium than for salts of zinc. The stability of a slurry of zeolite and salt and the amount by which the pH of a slurry is lowered by addition of a specific molar amount of a salt are indicators of the usefulness of that combination in the method of this invention. Consequently, the preferred second metals for use in the invention are aluminium, zirconium and tin.

The particle size of the zeolites used in this invention is adjusted to suit the intended use. Typically, the average particle size will be greater than 0.1 µm and, usually, less than 20 µm. More preferably, the zeolites will have an average particle size in the range 1 to 10 µm.

Various methods of assessing particle size are known and all give slightly different results. Some (e.g. the Sedigraph) give weight average particle sizes, some give number average particle sizes and some give volume average particle sizes. In the present invention, number average particle sizes are used, as measured by the Malvern Mastersizer^{™}. This equipment is also capable of giving a distribution of particle sizes from which it is possible to deduce the proportion of particles above or below any particle size.

The second component of the mixture used in the invention is a salt of a second metal as hereinbefore specified. The invention includes the use of mixtures in which more than one salt of a second metal (as defined) or salts of more than one second metal (as defined) are present. Suitable salts include halides, such as chlorides, nitrates, and, preferably, sulphates.

The method of the current invention comprises the use of the mixture of zeolite and salt of a second metal in the form of an aqueous composition at a pH in the range 4 to 10. Preferably, the aqueous composition has a pH in the range 5 to 9, and, more preferably, in the range 5 to 8.

The aqueous composition used in the invention can be prepared in a number of ways.

The zeolite, salt of a second metal, water and any other component used in the aqueous composition can be mixed in any order. Preferably, however, either the zeolite is added to an aqueous solution of the salt of a second metal or the zeolite and the salt of a second metal are simultaneously added to water or to an aqueous composition.

Simultaneous addition is particularly convenient and, consequently, a second embodiment of the invention comprises a powder comprising a mixture of
(a) a crystalline aluminosilicate represented by the empirical formula

   M_{2/n}O · Al₂O₃ · xSiO₂ · yH₂O

   wherein M represents a first metal moiety said first metal having a valency of n, x indicates the ratio of molecules of SiO₂ to molecules of Al₂O₃ and y indicates the ratio of molecules of water to molecules of Al₂O₃ and
(b) a salt of a second metal selected from the group consisting of Group III metals, metallic elements of Group IV, magnesium, titanium, chromium, iron, nickel, copper, zinc, zirconium and silver, said salt of a second metal being present in an amount which is sufficient to replace from about 2.0 to about 40 per cent of the first metal moiety, wherein in is an alkali metal.

Generally, this powder consists of a "dry" mixture in the sense that no water is added when the mixture is prepared. However, the zeolite is not normally anhydrous and, frequently, the salt of a second metal contains water of crystallisation. Therefore, the powder generally contains up to 25 per cent water, preferably from 5 to 22 per cent water. A particularly preferred powder comprises a mixture of zeolite A or zeolite P, containing from 10 to 22 per cent water by weight and aluminium sulphate hexadecahydrate.

Where the method of the invention involves the preparation of a "dry" mix which includes ingredients other than the zeolite and the metal salt (for example a detergent composition) and said "dry" mix is subsequently mixed with water, the zeolite and metal salt may be separately mixed with the other ingredients of the "dry" mix.

The invention makes possible the use of zeolites in detergent compositions which are used in a medium having a low pH. These detergent compositions are useful in fabric and dish washing and, in these applications, the aqueous composition is the wash liquor. Normally, the complete detergent composition is prepared before addition to the wash liquors and a further aspect of the invention is a detergent composition containing the powder comprising a mixture of zeolite and salt of a second metal as hereinbefore defined. The detergent composition may be of any physical type, for example, powder, liquid, gel, and solid bar. The zeolite may be used as the sole detergency builder in the detergent composition or it may be used with other builder materials. Suitable builder materials include polycarboxylate polymers, such as polyacrylates, acrylic-maleic copolymers or acrylic phosphinates, monomeric polycarboxylates such as nitrilotriacetates and ethylenediaminetetraacetates, inorganic salts such as sodium carbonate and other known detergency builders. If desired, a conventional phosphate builder can be added as a co-builder.

The detergent compositions will also contain, as essential ingredients, one or more detergent-active compounds, which may be chosen from soap and non-soap anionic, cationic, nonionic, amphoteric and zwitterionic, detergent-active compounds, and mixtures thereof. Suitable detergent-active compounds are described in the literature such as "Surface-Active Agents and Detergents" by Schwarz, Perry and Berch. The preferred detergent-active compounds are soaps and synthetic non-soap anionic and non-ionic compounds.

Suitable anionic compounds include alkylbenzene sulphonates, particularly sodium linear alkylbenzene sulphonates having an alkyl chain length of C₈ to C₁₅, primary and secondary alkyl sulphates, particularly sodium C₁₂ to C₁₅ primary alkyl sulphates, olefin sulphonates, alkane sulphonates, dialkyl sulphosuccinates and fatty acid ester sulphonates. Suitable nonionic surfactants include primary and secondary alcohol ethoxylates, especially the C₁₂ to C₁₅ primary and secondary alcohols ethoxylated with an average of from 3 to 20 moles of ethylene oxide per mole of alcohol.

The detergent compositions may also suitably contain a bleach system which, in dishwashing compositions, may, for example, be a chlorine bleach and, in fabric washing compositions, may be a peroxy bleach, such as an inorganic persalt or an organic peroxyacid, which may be employed in conjunction with an activator to improve the bleaching action.

Other materials that may be present in the detergent compositions include sodium silicate, fluorescers, antiredeposition agents, inorganic salts such as sodium sulphate, enzymes, lather control agents or lather boosters, as appropriate, pigments and perfumes.

The method of the invention includes the use of such detergent compositions for washing fabrics or dishes at a pH in the range 4 to 10, preferably 4 to 9. Consequently, one embodiment of the invention comprises washing fabrics or dishes with an aqueous mixture having a pH in the range 4 to 10, preferably 4 to 9 with a detergent composition comprising a mixture of
(a) a crystalline aluminosilicate represented by the empirical formula

   M_{2/n}O · Al₂O₃ · xSiO₂ · yH₂O

   wherein M represents a first metal moiety, said first metal having a valency of n, x indicates the ratio of molecules of SiO₂ to molecules of Al₂O₃ and y indicates the ratio of molecules of water to molecules of Al₂O₃ and
(b) a salt of a second metal selected from the group consisting of Group III metals, metallic elements of Group IV, magnesium, titanium, chromium, iron, nickel, copper, zinc, zirconium and silver, said salt of a second metal being present in an amount which is sufficient to replace from about 2.0 to about 40 per cent by weight of the first metal moiety.

A further method according to the invention is the use of a mixture of zeolite and salt of a second metal, as hereinbefore defined, in making paper. In this application, the aqueous composition will normally comprise a slurry containing at least cellulose pulp, zeolite and salt of a second metal. Normally, the zeolite is used to, at least partly, replace conventional fillers but the slurry may contain conventional fillers as well as other materials, such as pigments, dyes, sizes, starches and gums. Suitable pigments, and fillers include titanium dioxide, calcium carbonate, kaolin and coloured pigments.

The method of the invention includes the use of the mixture of zeolite and salt of a second metal in the manufacture of paper wherein an aqueous composition containing said mixture is used at a pH in the range 4 to 10, preferably 4 to 9. Typically, a slurry of the zeolite, salt of a second metal and cellulose pulp and other components as desired and having a pH in the range 4 to 9, is prepared, the slurry is formed into sheets, pressed, dried and, if desired, converted using conventional processes, to produce paper.

The aqueous composition used in the invention may be a dental composition, for example, in the form of a toothpaste, gel, cream or liquid, of the opaque, translucent or transparent variety. The dental composition contains the zeolite and the salt of a second metal as hereinbefore defined in addition to water and other conventional components of dental compositions. The zeolite in a dental composition will frequently replace conventionally used materials such as silicas, chalk and hydrated aluminas, but, such materials, and other conventional additives, such as calcium phosphate, calcium pyrophosphate, hydroxyapatites, insoluble metaphosphates etc. may also be present.

The dental composition may include one or more surfactants, preferably selected from anionic, non-ionic, amphoteric and zwitterionic surfactants, and mixtures thereof, all being suitable for dental and/or oral use. Suitable anionic surfactants may include soaps, alkyl sulphates, alkyl ether sulphates, alkaryl sulphonates, alkanoyl isethionates, alkanoyl taurates, alkyl succinates, alkyl sulphosuccinates, N-alkoyl sarcosinates, alkyl phosphates, alkyl ether phosphates, alkyl ether carboxylates and alpha-olefin sulphonates, especially their sodium, magnesium, ammonium and mono-, di- and triethanolamine salts. The alkyl and acyl groups generally contain from 8 to 18 carbon atoms and may be saturated. The alkyl ether sulphates, alkyl ether phosphates and alkyl ether carboxylates may contain from one to 10 ethylene oxide or propylene oxide units per molecule, and, preferably, 2 to 3 ethylene oxide units per molecule. Nonionic surfactants which may be suitable for use in the composition of the invention include sorbitan and polyglycerol esters of fatty acids, as well as ethylene oxide/propylene oxide block copolymers. Suitable amphoteric surfactants include betaines such as cocamidopropyl betaine and sulphobetaines.

The dental composition may also incorporate suitable well known polymer suspending or thickening agents such as polyacrylic acid, copolymers and cross-linked polymers of acrylic acid, copolymers of acrylic acid with a hydrophobic monomer, copolymers of carboxylic acid-containing monomers and acrylic esters, cross-linked copolymers of acrylic acid and acrylate esters, esters of ethylene glycol or esters of polyethylene glycol (e.g. fatty acid esters thereof), heteropolysaccharide gums such as xanthan and guar gums, and cellulose derivatives such as sodium carboxymethyl cellulose.

One or more other components that are conventionally found in an oral composition may be present in the dental composition and these include the following; flavoring substances such as peppermint, spearmint; artificial sweeteners; perfume or breath freshening substances; pearlescing agents; peroxy compounds such as hydrogen peroxide or peracetic acid; opacifiers; pigments and colourings; preservatives; moisturising agents; fluoride-containing compounds; anti-caries and anti-plaque agents; anti-tartar agents; anti-hypersensitivity agents; therapeutic agents such as zinc citrate, Triclosan (ex Ciba Geigy); proteins; enzymes; salts and baking soda.

The method of the invention includes the use of a mixture of zeolite, salt of a second metal as hereinbefore defined and water to prepare dental compositions as discussed. These dental compositions may be made by conventional methods of preparing such compositions. Pastes and creams may be prepared by conventional techniques, for example using high shear mixing systems under vacuum.

In view of the known therapeutic properties of zinc compounds, zinc is a useful second metal for use in the method of the invention, as applied to dental compositions.

The method of the invention is also useful in making aqueous paints. A further embodiment of the invention comprises an aqueous paint comprising a mixture of
(a) a crystalline aluminosilicate represented by the empirical formula

   M_{2/n}O · Al₂O₃ · xSiO₂ · yH₂O

   wherein M represents a first metal moiety, said first metal having a valency of n, x indicates the ratio of molecules of SiO₂ to molecutes of Al₂O₃ and y indicates the ratio of molecules of water to molecules of Al₂O₃ and
(b) a salt of a second metal selected from the group consisting of Group III metals, metallic elements of Group IV, magnesium, titanium, chromium, iron, nickel, copper, zinc, zirconium and silver, said salt of a second metal being present in an amount which is sufficient to replace from about 2.0 to about 40 per cent by weight of the first metal moiety, wherein mis and alkali metal

In addition the method of the invention is useful in adsorption processes and an embodiment of the invention comprises a process for adsorbing materials from an aqueous mixture wherein an adsorption agent comprises a mixture of
(a) a crystaline aluminosilicate represented by the empirical formula

   M_{2/n}O · Al₂O₃ · xSiO₂ · yH₂O

   wherein M represents a first metal moiety, said first metal having a valency of n, x indicates the ratio of molecules of SiO₂ to molecules of Al₂O₃ and y indicates the ratio of molecules of water to molecules of Al₂O₃ and
(b) a salt of a second metal selected from the group consisting of Group III metals, metallic elements of Group IV, magnesium, titanium, chromium, iron, nickel, copper, zinc, zirconium and silver, said salt of a second metal being present in an amount which is sufficient to replace from about 2.0 to about 40 per cent by weight of the first metal moiety, wherein M is an alkali metal

The method according to the invention can be a catalytic process. Therefore a further embodiment of the invention is a process catalysed by an aqueous mixture of
(a) a crystalline aluminosilicate represented by the empirical formula

   M_{2/n}O · Al₂O₃ · xSiO₂ · yH₂O

   wherein M represents a first metal moiety, said first metal having a valency of n, x indicates the ratio of molecules of SiO₂ to molecules of Al₂O₃ and y indicates the ratio of molecules of water to molecules of Al₂O₃ and
(b) a salt of a second metal selected from the group consisting of Group III metals, metallic elements of Group IV, magnesium, titanium, chromium, iron, nickel, copper, zinc, zirconium and silver, said salt of a second metal being present in an amount which is sufficient to replace from about 2.0 to about 40 per cent by weight of the first metal moiety, wherein M is an alkali metal.

The amount of zeolite present in the aqueous compositions used in the invention depends upon the actual application concerned and can vary widely. The amount of builder present in a detergent composition (dry powder) usually amounts to from 20 per cent to 80 per cent by weight and this may consist entirely of zeolite or co-builders may be present. Consequently, the amount of zeolite present may be from about 5 per cent to about 80 per cent by weight, typically up to 40 per cent by weight. Typically, detergents containing zeolites are added to wash liquors at a concentration in the range 1 to 5 g/l.

A paper typically contains from 2 to 40 per cent by weight filler in the final sheet and this may be all zeolite in paper prepared using the method of this invention. Typically, the final paper will contain from 0.5 to 20 per cent by weight of zeolite. The amount of zeolite present in the aqueous slurry of pulp and zeolite depends upon the type of paper which is being prepared, but the processes which are employed differ from conventional processes only in the substitution of the zeolite for other, conventional fillers.

Generally, dental compositions of use in the invention are aqueous compositions and will frequently contain from about 2 per cent up to about 20 per cent by weight of zeolite.

The zeolite used in the invention can be prepared by a conventional process. For example, a zeolite of type A can be prepared by mixing together sodium aluminate and sodium silicate at a temperature within the range of ambient temperature up to boiling point to form a gel, ageing the gel with stirring at a temperature usually in the range 70 to 95° C, separating the crystalline sodium aluminosilicate thus formed, washing, generally at a pH in the range 10 to 12.5, and drying. Zeolite of type P can be prepared by a similar process but zeolite type P formation is induced by the addition of type P seeds to the mixture of sodium aluminate and sodium silicate.

The stabilisation of a zeolite in an aqueous environment at a pH below 10 is demonstrated by mixing one or more salt of a second metal (as hereinbefore defined) and a zeolite in the form of a slurry at various concentrations. The pH of the resultant slurry is observed to decrease as the concentration of salt is increased. Surprisingly, the addition of aluminium sulphate (a weakly acidic salt) has a significantly more marked effect on the pH of the slurry than the addition of a strong acid such as sulphuric acid at the same molar concentration.

The invention is illustrated by the following, non-limiting examples.

### EXAMPLE 1

The effect of various salts on the pH of a slurry of the salts and a zeolite was determined by mixing different amounts of the salts with 5 g portions of zeolite Doucil^{®} A24 (P-type zeolite available from INEOS Silicas Ltd.) and slurrying the mixture in 45 mls of water followed by measurement of the pH of the slurry. The results, in which the salt concentrations are expressed as moles of salt per gram of zeolite, are given in Table 1 below.

**TABLE 1**

| **Salt** | **Concentration** **(Moles x 10⁻⁴)** | **pH of slurry** |
|---|---|---|
| MgSO₄ | 0 | 11.3 |
| | 1.67 | 9.8 |
| | 4.17 | 9.2 |
| | 8.33 | 8.9 |
| | 16.7 | 8.7 |
| ZnSO₄.7H₂O | 0 | 11.3 |
| | 8.7 | 9.2 |
| | 13.9 | 8.6 |
| | 27.8 | 7.6 |
| | 34.8 | 7.2 |
| SnCl₄.5H₂O | 0 | 11.3 |
| | 1.42 | 7.6 |
| | 2.85 | 6.4 |
| | 5.7 | 4.6 |
| Al₂(SO₄)₃.16H₂O | 0 | 11.3 |
| | 0.32 | 9.1 |
| | 0.79 | 7.8 |
| | 1.6 | 6.5 |
| | 3.2 | 5.4 |
| FeSO₄.7H₂O | 0 | 11.3 |
| | 1.8 | 8.8 |
| | 3.6 | 8.0 |
| | 7.2 | 7.4 |
| Zr(SO₄)₂.2H₂O | 0 | 11.3 |
| | 1.7 | 7.8 |
| | 3.4 | 6.4 |
| | 5.7 | 5.4 |

In contrast to the effect on pH of the salt solutions shown in Table 1 above, a slurry of 5 g Doucil^{®} A24 in 50 ml water was diluted progressively with portions of water up to a slurry containing 1950 ml water and the final pH was found to be 10.6.

The slurry containing 3.2 x 10⁻⁴ moles of Al₂(SO₄)₃ was kept for 3 weeks. After three weeks storage, the slurry was filtered, washed and dried. The dried product was subjected to X-ray analysis and no significant structural damage could be detected in the zeolite.

### EXAMPLE 2

Slurries containing aluminium sulphate at concentrations the same as those given in Table 1 above were prepared except that the salt was first dissolved in water and the zeolite was added to the salt solution. The pH of the resultant slurries were measured and found to be identical to those listed in Table 1.

### EXAMPLE 3

Example 1 was repeated using aluminium sulphate except that zeolite 4A was used instead of Doucil^{®} A24. The pH values of the resultant slurries are given in Table 2 below.

**TABLE 2**

| **Concn. of Al₂(SO₄)₃** **(Moles x 10⁻⁴)** | **pH of slurry** |
|---|---|
| 0 | 11.2 |
| 0.32 | 9.1 |
| 0.79 | 7.8 |
| 1.6 | 6.5 |
| 3.2 | 5.4 |

### EXAMPLE 4

A detergent composition suitable for use in the method of the invention is as follows.

| | **Weight %** |
|---|---|
| Sodium Linear Alkylbenzene Sulphonate | 24.0 |
| Nonionic Surfactant | 2.0 |
| Sodium Stearate | 1.0 |
| Zeolite (Doucil^{®} A24) | 35.0 |
| Aluminium Sulphate | 5.0 |
| Sodium Carbonate | 15.0 |
| Sodium Sulphate | 5.0 |
| Water and minor ingredients | 18.0 |

The zeolite and aluminium sulphate were mixed before addition to the other ingredients.

This composition was added to water as a 5% by weight slurry and the slurry was found to have a pH of 9.0. In comparison, a similar composition, without the aluminium sulphate had a pH of 11.1. This change in pH represents a change in concentration of hydrogen ions of two orders of magnitude.

## Claims

1. A method of preparing an aqueous composition with a pH in the range 4 to 10 comprising forming a mixture of
(a) a crystalline aluminosilicate represented by the empirical formula
M_{2/n}O · Al₂O₃ · xSiO₂ · yH₂O
wherein M represents a first metal moiety, said first metal having a valency of n, x indicates the ratio of molecules of SiO₂ to molecules of Al₂O₃ and y indicates the ratio of molecule of water to molecules of Al₂O₃ and
(b) a salt of a second metal selected from the group consisting of Group III metals, metallic elements of Group IV, magnesium, titanium, chromium, iron, nickel, copper, zinc, zirconium and silver, said salt of a second metal being present in an amount which is sufficient to replace from about 2.0 to about 40 per cent by weight of the first metal moiety,
and using said mixture in the aqueous composition, **characterised in that** M is an alkali metal

2. A method according to claim 1 **characterised in that** the aluminosilicate is zeolite P, zeolite X or zeolite A.

3. A method according to any one of the preceding claims **characterised in that** the second metal is aluminium or tin.

4. A method according to any one of the preceding claims **characterised in that** the aluminosilicate is a zeolite having a particle size in the range 0.1 µm to 20 µm.

5. A method according to any one of the preceding claims **characterised in that** the aqueous composition has a pH in the range 4 to 9.

6. A method according to any one of the preceding claims **characterised in that** the mixture of aluminosilicate and second metal salt is used in the form of a detergent composition.

7. A method according to any one of claims 1 to 6 **characterised in that** the aqueous composition is used in the production of paper.

8. A method according to any one of claims 1 to 6 **characterised in that** the aqueous composition is a dental composition.

9. A method according to any one of claims 1 to 6 **characterised in that** the aqueous composition is a paint.

10. A powder comprising a mixture of
(a) a crystalline aluminosilicate represented by the empirical formula
M_{2/n}O · Al₂O₃ · xSiO₂ · yH₂O
wherein M represents a first metal moiety said first metal having a valency of n, x indicates the ratio of molecules of SiO₂ to molecules of Al₂O₃ and y indicates the ratio of molecules of water to molecules of Al₂O₃ and
(b) a salt of a second metal selected from the group consisting of Group III metals, metallic elements of Group IV. magnesium, titanium, chromium, iron, nickel, copper, zinc, zirconium and silver, said salt of a second metal being present in an amount which is sufficient to replace from about 2.0 to about 40 per cent by weight of the first metal moiety, wherein M is an alkali metal.

11. A powder according to claim 10 **characterised in that** up to 25 per cent by weight water is present.

12. The use, in the manufacture of paper from an aqueous slurry of cellulose pulp at a pH in the range 4 to 10, of a mixture of
(a) a crystalline aluminosilicate represented by the empirical formula
M_{2/n}O · Al₂O₃ · xSiO₂ · yH₂O
wherein M represents a first metal moiety, said first metal having a valency of n, x indicates the ratio of molecules of SiO₂ to molecules of Al₂O₃ and y indicates the ratio of molecules of water to molecules of Al₂O₃ and
(b) a salt of a second metal selected from the group consisting of Group III metals, metallic elements of Group IV, magnesium, titanium, chromium, iron, nickel, copper, zinc, zirconium and silver, said salt of a second metal being present in an amount which is sufficient to replace from about 2.0 to about 40 per cent by weight of the first metal moiety, wherein M is an alkali metal

13. Use according to claim 12 in which the aqueous slurry of cellulose pulp is at a pH in the range 4 to 9.

14. The use, in the manufacture of a dental composition, of a powder as claimed in claim 10 or claim 11.

15. Use according to claim 14 in which the dental composition is a toothpaste.

16. A dental composition comprising an aqueous composition at a pH in the range 4 to 10 containing a mixture of
(a) a crystalline aluminosilicate represented by the empirical formula
M_{2/n}O · Al₂O₃ · xSiO₂ · yH₂O
wherein M represents a first metal moiety, said first metal having a valency of n, x indicates the ratio of molecules of SiO₂ to molecules of Al₂O₃ and y indicates the ratio of molecules of water to molecules of Al₂O₃ and
(b) a salt of a second metal selected from the group consisting of Group III metals, metallic elements of Group IV, magnesium, titanium, chromium, iron, nickel, copper, zinc, zirconium and silver, said salt of a second metal being present in an amount which is sufficient to replace from about 2.0 to about 40 per cent by weight of the first metal moiety, wherein M is an alkali metal

17. A dental composition according to claim 16 in the form of a toothpaste.

18. A method of manufacturing a dental composition comprising forming a mixture of a crystalline aluminosilicate represented by the empirical formula
M_{2/n}O · Al₂O₃ · xSiO₂ · yH₂O
wherein M represents a first metal moiety, said first metal having a valency of n, x indicates the ratio of molecules of SiO₂ to molecules of Al₂O₃ and y indicates the ratio of molecules of water to molecules of Al₂O₃ and (b) a salt of a second metal selected from the group consisting of Group III metals, metallic elements of Group IV, magnesium, titanium, chromium, iron, nickel, copper, zinc, zirconium and silver, said salt of a second metal being present in an amount which is sufficient to replace from about 2.0 to about 40 per cent by weight of the first metal moiety, wherein M is an alkali metal,
and using said mixture in an aqueous dental composition at a pH in the range 4 to 10.

19. A method according to claim 18 in which the dental composition is a toothpaste.

20. A method according to claim 18 or claim 19 in which the mixture is a powder.

21. A method according to claim 20 in which up to 25 per cent by weight water is present in the powder.

## Patentansprüche

1. Verfahren zum Herstellen einer wässerigen Zusammensetzung mit einem pH in dem Bereich 4 bis 10, umfassend Erzeugen eines Gemisches von
(a) einem kristallinen Aluminosilicat, dargestellt durch die empirische Formel
M_{2/n}O • Al₂O₃ • xSiO₂ • yH₂O
wobei M eine erste Metalleinheit darstellt, wobei das erste Metall eine Valenz von n hat, x das Verhältnis von Molekülen von SiO₂ zu Molekülen von Al₂O₃ anzeigt und y das Verhältnis von Molekülen von Wasser zu Molekülen von Al₂O₃ anzeigt, und
(b) einem Salz eines zweiten Metalls, ausgewählt aus der Gruppe, bestehend aus Metallen der Gruppe III, metallischen Elementen der Gruppe IV, Magnesium, Titan, Chrom, Eisen, Nickel, Kupfer, Zink, Zirconium und Silber, wobei das Salz eines zweiten Metalls in einer Menge vorhanden ist, welche ausreichend ist, um von etwa 2,0 bis etwa 40 Gewichtsprozent der ersten Metalleinheit zu ersetzen,
und Verwenden des Gemisches in der wässerigen Zusammensetzung, **dadurch gekennzeichnet, daß** M ein Alkalimetall ist.

2. Verfahren gemäß Anspruch 1, **dadurch gekennzeichnet, daß** das Aluminosilicat Zeolith P, Zeolith X oder Zeolith A ist.

3. Verfahren gemäß einem der vorhergehenden Ansprüche, **dadurch gekennzeichnet, daß** das zweite Metall Aluminium oder Zinn ist.

4. Verfahren gemäß einem der vorhergehenden Ansprüche, **dadurch gekennzeichnet, daß** das Aluminosilicat ein Zeolith mit einer Teilchengröße in dem Bereich 0,1 µm bis 20 µm ist.

5. Verfahren gemäß einem der vorhergehenden Ansprüche, **dadurch gekennzeichnet, daß** die wässerige Zusammensetzung einen pH in dem Bereich 4 bis 9 hat.

6. Verfahren gemäß einem der vorhergehenden Ansprüche, **dadurch gekennzeichnet, daß** das Gemisch von Aluminosilicat und dem zweiten Metallsalz in der Form einer Detergenszusammensetzung verwendet wird.

7. Verfahren gemäß einem der Ansprüche 1 bis 6, **dadurch gekennzeichnet, daß** die wässerige Zusammensetzung in der Herstellung von Papier verwendet wird.

8. Verfahren gemäß einem der Ansprüche 1 bis 6, **dadurch gekennzeichnet, daß** die wässerige Zusammensetzung eine dentale Zusammensetzung ist.

9. Verfahren gemäß einem der Ansprüche 1 bis 6, **dadurch gekennzeichnet, daß** die wässerige Zusammensetzung ein Anstrich ist.

10. Pulver, umfassend ein Gemisch von
(a) einem kristallinen Aluminosilicat, dargestellt durch die empirische Formel
M_{2/n}O • Al₂O₃ • xSiO₂ • yH₂O wobei M eine erste Metalleinheit darstellt, wobei das erste Metall eine Valenz von n hat, x das Verhältnis von Molekülen von SiO₂ zu Molekülen von Al₂O₃ anzeigt und y das Verhältnis von Molekülen von Wasser zu Molekülen von Al₂O₃ anzeigt, und
(b) einem Salz eines zweiten Metalls, ausgewählt aus der Gruppe, bestehend aus Metallen der Gruppe III, metallischen Elementen der Gruppe IV, Magnesium, Titan, Chrom, Eisen, Nickel, Kupfer, Zink, Zirconium und Silber, wobei das Salz eines zweiten Metalls in einer Menge vorhanden ist, welche ausreichend ist, um von etwa 2,0 bis etwa 40 Gewichtsprozent der ersten Metalleinheit zu ersetzen, wobei M ein Alkalimetall ist.

11. Pulver gemäß Anspruch 10, **dadurch gekennzeichnet, daß** bis zu 25 Gewichtsprozent Wasser vorhanden sind.

12. Verwendung in der Herstellung von Papier aus einer wässerigen Aufschlämmung von Cellulosebrei mit einem pH in dem Bereich 4 bis 10 eines Gemisches von
(a) einem kristallinen Aluminosilicat, dargestellt durch die empirische Formel
M_{2/n}O • Al₂O₃ • xSiO₂ • yH₂O
wobei M eine erste Metalleinheit darstellt, wobei das erste Metall eine Valenz von n hat, x das Verhältnis von Molekülen von SiO₂ zu Molekülen von Al₂O₃ anzeigt und y das Verhältnis von Molekülen von Wasser zu Molekülen von Al₂O₃ anzeigt, und
(b) einem Salz eines zweiten Metalls, ausgewählt aus der Gruppe, bestehend aus Metallen der Gruppe III, metallischen Elementen der Gruppe IV, Magnesium, Titan, Chrom, Eisen, Nickel, Kupfer, Zink, Zirconium und Silber, wobei das Salz eines zweiten Metalls in einer Menge vorhanden ist, welche ausreichend ist, um von etwa 2,0 bis etwa 40 Gewichtsprozent der ersten Metalleinheit zu ersetzen, wobei M ein Alkalimetall ist.

13. Verwendung gemäß Anspruch 12, bei welcher die wässerige Aufschlämmung von Cellulosebrei bei einem pH in dem Bereich 4 bis 9 ist.

14. Verwendung in der Herstellung einer dentalen Zusammensetzung eines Pulvers, wie es in Anspruch 10 oder Anspruch 11 beansprucht ist.

15. Verwendung gemäß Anspruch 14, bei welcher die dentale Zusammensetzung eine Zahnpasta ist.

16. Dentale Zusammensetzung, umfassend eine wässerige Zusammensetzung mit einem pH in dem Bereich 4 bis 10, enthaltend ein Gemisch von
(a) einem kristallinen Aluminosilicat, dargestellt durch die empirische Formel
M_{2/n}O • Al₂O₃ • XSiO₂ • yH₂O
wobei M eine erste Metalleinheit darstellt, wobei das erste Metall eine Valenz von n hat, x das Verhältnis von Molekülen von SiO₂ zu Molekülen von Al₂O₃ anzeigt und y das Verhältnis von Molekülen von Wasser zu Molekülen von Al₂O₃ anzeigt, und
(b) einem Salz eines zweiten Metalls, ausgewählt aus der Gruppe, bestehend aus Metallen der Gruppe III, metallischen Elementen der Gruppe IV, Magnesium, Titan, Chrom, Eisen, Nickel, Kupfer, Zink, Zirconium und Silber, wobei das Salz eines zweiten Metalls in einer Menge vorhanden ist, welche ausreichend ist, um von etwa 2,0 bis etwa 40 Gewichtsprozent der ersten Metalleinheit zu ersetzen, wobei M ein Alkalimetall ist.

17. Dentale Zusammensetzung gemäß Anspruch 16 in der Form einer Zahnpasta.

18. Verfahren zum Herstellen einer dentalen Zusammensetzung, umfassend Erzeugen eines Gemisches von
(a) einem kristallinen Aluminosilicat, dargestellt durch die empirische Formel
M_{2/n}O • Al₂O₃ • xSiO₂ • yH₂O
wobei M eine erste Metalleinheit darstellt, wobei das erste Metall eine Valenz von n hat, x das Verhältnis von Molekülen von SiO₂ zu Molekülen von Al₂O₃ anzeigt und y das Verhältnis von Molekülen von Wasser zu Molekülen von Al₂O₃ anzeigt, und
(b) einem Salz eines zweiten Metalls, ausgewählt aus der Gruppe, bestehend aus Metallen der Gruppe III, metallischen Elementen der Gruppe IV, Magnesium, Titan, Chrom, Eisen, Nickel, Kupfer, Zink, Zirconium und Silber, wobei das Salz eines zweiten Metalls in einer Menge vorhanden ist, welche ausreichend ist, um von etwa 2,0 bis etwa 40 Gewichtsprozent der ersten Metalleinheit zu ersetzen, wobei M ein Alkalimetall ist,
und Verwenden des Gemisches in einer wässerigen dentalen Zusammensetzung mit einem pH in dem Bereich 4 bis 10.

19. Verfahren gemäß Anspruch 18, bei welchem die dentale Zusammensetzung eine Zahnpasta ist.

20. Verfahren gemäß Anspruch 18 oder Anspruch 19, bei welchem das Gemisch ein Pulver ist.

21. Verfahren gemäß Anspruch 20, bei welchem bis zu 25 Gewichtsprozent Wasser in dem Pulver vorhanden sind.

## Revendications

1. Procédé pour la préparation d'une composition aqueuse avec un pH dans l'intervalle de 4 à 10 comprenant la formation d'un mélange:
(a) d'un aluminosilicate cristallin représenté par la formule empirique:
M_{2/n}O • AL₂O₃ • xSiO₂ • yH₂O
dans laquelle M représente une première fraction de métal, ledit premier métal possédant une valence de n, x indique le rapport des molécules de SiO₂ sur les molécules de Al₂O₃ et y indique le rapport des molécules d'eau sur les molécules de Al₂O₃ et
(b) d'un sel d'un deuxième métal choisi dans le groupe constitué de métaux du Groupe III, d'éléments métalliques du Groupe IV, de magnésium, de titane, de chrome, de fer, de nickel, de cuivre, de zinc, de zirconium et d'argent, ledit sel d'un deuxième métal étant présent dans une quantité qui est suffisante pour remplacer d'environ 2,0 à environ 40 pour cent en poids de la première fraction de métal,
et l'utilisation dudit mélange dans la composition aqueuse, **caractérisé en ce que** M est un métal alcalin.

2. Procédé selon la revendication 1, **caractérisé en ce que** l'aluminosilicate est une zéolite P, une zéolite X ou une zéolite A.

3. Procédé selon l'une quelconque des revendications précédentes, **caractérisé en ce que** le deuxième métal est l'aluminium ou l'étain.

4. Procédé selon l'une quelconque des revendications précédentes, **caractérisé en ce que** l'aluminosilicate est une zéolite possédant une taille de particules dans l'intervalle de 0,1 µm à 20 µm.

5. Procédé selon l'une quelconque des revendications précédentes, **caractérisé en ce que** la composition aqueuse possède un pH dans l'intervalle de 4 à 9.

6. Procédé selon l'une quelconque des revendications précédentes, **caractérisé en ce que** le mélange d'aluminosilicate et de deuxième sel métallique est utilisé sous la forme d'une composition de détergent.

7. Procédé selon l'une quelconque des revendications 1 à 6, **caractérisé en ce que** la composition aqueuse est utilisée dans la production de papier.

8. Procédé selon l'une quelconque des revendications 1 à 6, **caractérisé en ce que** la composition aqueuse est une composition dentaire.

9. Procédé selon l'une quelconque des revendications 1 à 6, **caractérisé en ce que** la composition aqueuse est une peinture.

10. Poudre comprenant un mélange:
(a) d'un aluminosilicate cristallin représenté par la formule empirique:
M_{2/n}O • Al₂O₃ • xSiO₂ • yH₂O
dans laquelle M représente une première fraction de métal, ledit premier métal possédant une valence de n, x indique le rapport des molécules de SiO₂ sur les molécules de Al₂O₃ et y indique le rapport des molécules d'eau sur les molécules de Al₂O₃ et
(b) d'un sel d'un deuxième métal choisi dans le groupe constitué de métaux du Groupe III, d'éléments métalliques du Groupe IV, de magnésium, de titane, de chrome, de fer, de nickel, de cuivre, de zinc, de zirconium et d'argent, ledit sel d'un deuxième métal étant présent dans une quantité qui est Suffisante pour remplacer d'environ 2,0 à environ 40 pour cent en poids de la première fraction de métal, où M est un métal alcalin.

11. Poudre selon la revendication 10, **caractérisée en ce qu'**il est présent jusqu'à 25 pour cent en poids d'eau.

12. Utilisation, dans la fabrication d'un papier à partir d'une suspension épaisse aqueuse de pâte de cellulose à un pH dans l'intervalle de 4 à 10, d'un mélange:
(a) d'un aluminosilicate cristallin représenté par la formule empirique:
M_{2/n}O • Al₂O₃ • xSiO₂ • yH₂O
dans laquelle M représente une première fraction de métal, ledit premier métal possédant une valence de n, x indique le rapport des molécules de SiO₂ sur les molécules de Al₂O₃ et y indique le rapport des molécules d'eau sur les molécules de Al₂O₃ et
(b) d'un sel d'un deuxième métal choisi dans le groupe constitué de métaux du Groupe III, d'éléments métalliques du Groupe IV, de magnésium, de titane, de chrome, de fer, de nickel, de cuivre, de zinc, de zirconium et d'argent, ledit sel d'un deuxième métal étant présent dans une quantité qui est suffisante pour remplacer d'environ 2,0 à environ 40 pour cent en poids de la première fraction de métal, où M est un métal alcalin.

13. Utilisation selon la revendication 12, dans laquelle la suspension épaisse aqueuse de pâte de cellulose est à un pH dans l'intervalle de 4 à 9.

14. Utilisation, dans la fabrication d'une composition dentaire, d'une poudre telle que revendiquée dans la revendication 10 ou la revendication 11.

15. Utilisation selon la revendication 14, dans laquelle la composition dentaire est un dentifrice.

16. Composition dentaire comprenant une composition aqueuse à un pH dans l'intervalle de 4 à 10 contenant un mélange:
(a) d'un aluminosilicate cristallin représenté par la formule empirique:
M_{2/n}O • Al₂O₃ • xSiO₂ •yH₂O
dans laquelle M représente une première fraction de métal, ledit premier métal possédant une valence de n, x indique le rapport des molécules de SiO₂ sur les molécules de Al₂O₃ et y indique le rapport des molécules d'eau sur les molécules de Al₂O₃ et
(b) d'un sel d'un deuxième métal choisi dans le groupe constitué de métaux du Groupe III, d'éléments métalliques du Groupe IV, de magnésium, de titane, de chrome, de fer, de nickel, de cuivre, de zinc, de zirconium et d'argent, ledit sel d'un deuxième métal étant présent dans une quantité qui est suffisante pour remplacer d'environ 2,0 à environ 40 pour cent en poids de la première fraction de métal, où M est un métal alcalin.

17. Composition dentaire selon la revendication 16 sous la forme d'un dentifrice.

18. Procédé pour la fabrication d'une composition dentaire comprenant la formation d'un mélange:
(a) d'un aluminosilicate cristallin représenté par la formule empirique:
M_{2/n}O • Al₂O₃ • xSiO₂ • yH₂O
dans laquelle M représente une première fraction de métal, ledit premier métal possédant une valence de n, x indique le rapport des molécules de SiO₂ sur les molécules de Al₂O₃ et y indique le rapport des molécules d'eau sur les molécules de Al₂O₃ et
(b) d'un sel d'un deuxième métal choisi dans le groupe constitué de métaux du Groupe III, d'éléments métalliques du Groupe IV, de magnésium, de titane, de chrome, de fer, de nickel, de cuivre, de zinc, de zirconium et d'argent, ledit sel d'un deuxième métal étant présent dans une quantité qui est suffisante pour remplacer d'environ 2,0 à environ 40 pour cent en poids de la première fraction de métal, où M est un métal alcalin, et l'utilisation dudit mélange dans une composition dentaire aqueuse à un pH dans l'intervalle de 4 à 10.

19. Procédé selon la revendication 18, dans lequel la composition dentaire est un dentifrice.

20. Procédé selon la revendication 18 ou la revendication 19, dans lequel le mélange est une poudre.

21. Procédé selon la revendication 20, dans lequel il est présent jusqu'à 25 pour cent en poids d'eau dans la poudre.
